# EUROPEAN PATENT SPECIFICATION

(11) **EP 1 013 635 B1**
(45) Date of publication and mention of the grant of the patent: **18.09.2002**
(21) Application number: 99309985.2
(22) Date of filing: 10.12.1999
(51) Int. Cl.: C07C 201/12, C07C 205/38

(54) **Preparation of diphenylethers**
Herstellung von Diphenylethern
Préparation d' éthers diphenyliques

(30) Priority: 23.12.1998 US 113934 P
(43) Date of publication of application: 28.06.2000
(73) Proprietor: Dow AgroSciences LLC, Indianapolis, Indiana 46268 (US)
(72) Inventor: Roemmele, Renee Caroline, Maple Glen, Pennsylvania 19002 (US); Kelly, Martha Jean, Norristown, Pennsylvania 19403 (US)
(74) Representative: Huber, Bernhard, Dipl.-Chem.

(56) References cited:
- EP-A- 0 352 563
- CH-A- 567 359
- MAKOSZA M ET AL: "Hydroxylation of Nitroarenes with Alkyl Hydroperoxide Anions via Vicarious Nucleophilic Substitution of Hydrogen" J. ORG. CHEM. (JOCEAH,00223263);1998; VOL.63 (13); PP.4199-4208, XP002131334 Polish Academy of Sciences;Institute of Organic Chemistry; Warsaw; 01-224; Pol. (PL)

## Description

The present invention is concerned with the preparation of 3-hydroxy diphenylethers and with the use of such 3-hydroxy diphenylethers in the preparation of 3-alkoxy diphenylethers. More particularly, the present invention is concerned with the preparation of 3-hydroxy-4-nitrodiphenylethers and with the use of such 3-hydroxy-4-nitrodiphenylethers in the preparation of 3-alkoxy-4-nitrodiphenylethers.

Many 3-hydroxy-4-nitrodiphenylethers and 3-alkoxy-4-nitrodiphenylethers are known to be herbicidally active and methods for the preparation of such compounds are disclosed in, for example, GB-A-1,423,376, US-A-4,358,308, US-A-4,419,122, US-A-4,419,124, US-A-4,220,468 and US-A-4,415,354. These documents disclose a method for preparing 3-hydroxy-4-nitrodiphenylethers and 3-alkoxy-4-nitrodiphenylethers which involves reacting a suitably substituted phenol, or the potassium or sodium salt of such a phenol, with a suitably substituted halobenzene in the presence of an alkaline agent. CH-A-567 359 discloses 3-hydroxy-4-nitro diphenyl ethers which are prepared by nitration of the acetoxy substituted diphenyl ether compound.
Certain of these documents, for example US-A-4,220,468 and US-A-4,415,354, also disclose the preparation of 3-substituted alkoxy-4-nitrodiphenylethers by a process involving the reaction of the corresponding diphenylether precursor in which the 3-substituent is a good leaving group, such as halo, with an appropriately substituted alcohol. However, a disadvantage of such processes for producing the diphenylethers is that a mixture of products is generally obtained and this reduces the yield of the desired product and requires purification in order to obtain the desired product.

An alternative to the above known procedure for preparing 3-hydroxy-4-nitrodiphenylethers and 3-alkoxy-4-nitrodiphenylethers would be to introduce the hydroxy or alkoxy group directly into the 3-position in an appropriate 4-nitrodiphenylether (i.e. to introduce the hydroxy or alkoxy group ortho to the nitro group). However, it is known in the art that, due to the presence of two aromatic rings in a diphenylether, it is very difficult to selectively functionalize one aromatic ring over the other and that functionalization of such diphenylethers often results in a mixture of isomers. The production of such a mixture of isomers is disadvantageous, firstly, because it reduces the yield of the desired isomer and, secondly, because purification of the mixture of isomers is needed in order to obtain the desired isomer, the purification giving rise to further losses in yield of the desired isomer, often being complex, and generating waste material that must be disposed of.

Further, M. Makosza and J. Winiarski, J. Acc. Chem. Res., 1987, (20), pages 282-289, M. Makosza and K. Sienkiewicz, J. Organic Chemistry, (55), 1990, pages 4979-4981, M. Makosza and S. Sienkiewicz, J. Organic Chemistry, (63), 1998, pages 4199-4208 and EP-A-0 353 563 disclose vicarious nucleophilic substitution of aryl compounds. For example, M. Makosza and K. Sienkiewicz, J. Organic Chemistry, (55), 1990, pages 4979-4981 discloses vicarious nucleophilic substitution to insert a hydroxy group into an aryl ring of a nitroarene to form ortho and/or para nitrophenols. However, due to the significant difference in structure between the nitroarene compounds disclosed in the M. Makosza and K. Sienkiewicz article, which contain only a single aromatic ring, and the much larger diphenylether compounds with which the present invention is concerned, it would not be expected that nucleophilic substitution of a hydroxy group into one of the positions ortho to a nitro group in the diphenylether compounds would occur.

We have, however, now unexpectedly found that substitution of a hydroxy group into one of the positions ortho to a nitro group in 4-nitrodiphenylether compounds does occur, and occurs with a high degree of selectivity.

Accordingly, in a first aspect of the present invention there is provided a process for preparing a compound of formula (I): 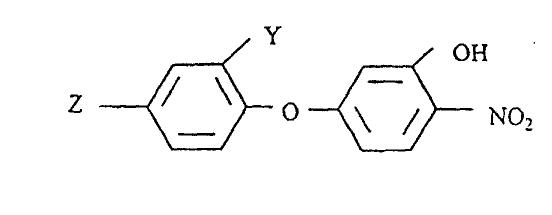 wherein
Y is alkyl, halogen or a trihaloalkyl group, preferably halogen or a trihaloalkyl group, and
Z is alkyl, halogen or a trihaloalkyl , preferably, halogen or a trihaloalkyl group,
and
the process comprising reacting a compound of formula (II): 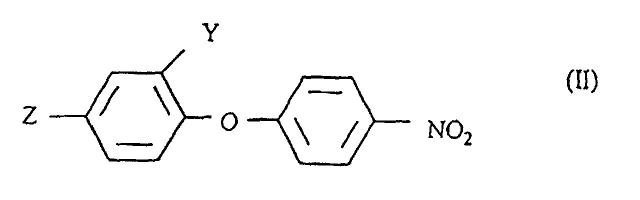 wherein Z and Y are as defined above, with a hydroperoxide in the presence of liquid ammonia and a strong base and, optionally, in an organic solvent.

As used herein, the term "alkyl" refers to straight and branched chain alkyl groups containing from one to twenty carbon atoms, preferably containing from one to six carbon atoms.

As used herein, the term "trihaloalkyl" refers to straight and branched chain trihaloalkyl groups containing from one to twenty carbon atoms, preferably containing from one to four carbon atoms.

As used herein, "halogen" and "halo" refer to fluorine, chlorine, bromine and iodine.

Preferably, Y is halogen and, more preferably, Y is chlorine.

Preferably, Z is trifluoroalkyl and, more preferably, Z is trifluoromethyl.

Examples of suitable hydroperoxides include tertiary butyl hydroperoxide and cumylhydroperoxide, with tertiary butylhydroperoxide being preferred.

Suitable strong bases are, for example, alkali metal alkoxides such as alkali metal methoxides and butoxides. Preferably, the strong base is potassium tertiary butoxide

Preferably, the strong base is used in an amount of from 2 to 20 equivalents, more preferably 5 to 10 equivalents per equivalent of the compound of formula (II).

The ammonia is used in the form of liquid ammonia. If the reactants, other than the liquid ammonia, are soluble in the liquid ammonia, then the reaction may be conducted in the absence of added organic solvent.

Examples of suitable organic solvents include hydrocarbons such as hexane, chlorinated hydrocarbons such as chloroform and dichloromethane, ethers such as aliphatic ethers and tetrahydrofuran. An example of a suitable aliphatic ether is diethylether. Preferably, the ether is tetrahydrofuran.

Preferably the reaction to produce the compound of formula (I) is carried out at a temperature of from -50°C to 0°C.

The reaction may, for example, be carried out at a pressure of 1 to 5 atmospheres. When the reaction temperature is above -33°C, the reaction is carried out at a pressure above atmospheric pressure, for example at a pressure of up to 5 atmospheres, such that the ammonia is in liquid form.

The 3-hydroxy-4-nitrodiphenylether of formula (I) may be alkylated to produce the corresponding 3-alkoxy-4-nitrodiphenylether.

Accordingly, in a second aspect of the present invention there is provided a process for producing a compound of formula (III): 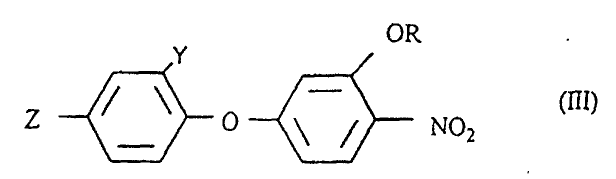 wherein R is an alkyl group or a substituted alkyl group, and Y and Z are as defined above, which comprises reacting a compound of formula (II): 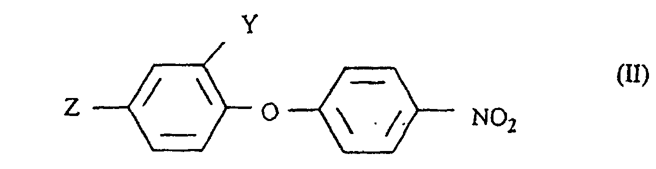 wherein Y and Z are as defined above, with a hydroperoxide in the presence of liquid ammonia and a strong base and, optionally, in an organic solvent, to produce a compound of formula (I): 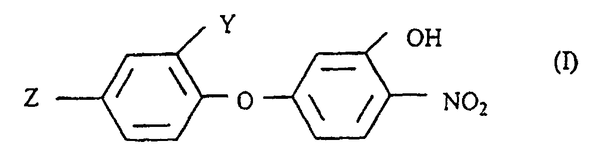 wherein Y and Z are as defined above, and alkylating a compound of formula (I) to produce a corresponding compound of formula (III).

In a preferred embodiment of the second aspect of the present invention
Y is alkyl, halogen or a trihaloalkyl group, preferably halogen or a trihaloalkyl group, and
Z is alkyl, halogen or a trihaloalkyl group, preferably halogen or a trihaloalkyl group.

Preferably, R is an alkyl group, and more preferably, R is ethyl.

As used herein, the term "substituted alkyl group" refers to straight and branched chain alkyl groups containing from one to twenty carbon atoms, preferably containing one to six carbon atoms. In such substituted alkyl groups the alkyl groups may, for example, be substituted with one or more substituents selected from: halo; hydroxy; alkoxy, preferably (C₁-C₄)alkoxy; alkoxycarbonyl, preferably (C₁-C₄)alkoxycarbonyl; carboxyl; alkylcarbonyl, preferably (C₁-C₄)alkylcarbonyl; alkylthio, preferably (C₁-C₄)alkylthio; alkylsulfonyl, preferably (C₁-C₄)alkylsulfonyl; epoxy; aminocarbonyl; alkylamino, preferably (C₁-C₄)alkylamino; and alkylaminocarbonyl, preferably (C₁-C₄)ahalkylaminocarbonyl.

Any known alkylating method may be used to produce the compound of formula (III). For example, the alkylation may comprise reacting a compound of formula (I) with a strong base and an alkylating agent of formula RX wherein R is as defined above and X is halogen. Preferably, the compound of formula (I) is reacted with the strong base followed by reaction with the alkylating agent of formula RX. It is believed that the base reacts with the -OH group of the compound of formula (I) to produce the salt, and this salt reacts with the compound of formula RX to form the compound of formula (III). Sufficient strong base should, therefore, preferably be added to form the salt of the compound of formula (I).

Preferably, X is chlorine, bromine or iodine.

Examples of suitable strong bases for use in the above process for preparing the compound of formula (III) include alkali metal hydroxides such as sodium hydroxide, alkali metal hydrides such as sodium hydride, and alkoxides such as alkali metal hydroxides. Examples of suitable alkali metal alkoxides include sodium methoxide and potassium t-butoxide.

The alkylation reaction is preferably conducted in the presence of an organic solvent. Examples of suitable solvents are aprotic solvents such as sulfolane, dimethylsulfoxide and N,N-dimethylformamide, and preferably sulfolane.

The compound of formula (II) may be prepared by known processes, for example, by a process comprising reacting a compound of formula (IV): 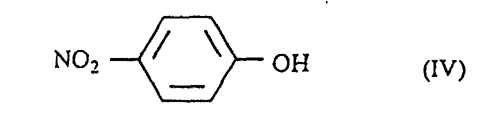 with a compound of formula (V): 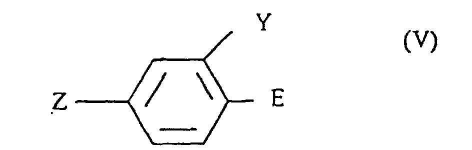 wherein:
E is halogen, for example chloro or fluoro, preferably fluoro, and
Y and Z are as defined above,
in the presence of a strong base.

The above preparation of the compound of formula (II) is preferably carried out in the presence of a solvent such as dimethylsulfoxide or sulfolane, preferably dimethylsulfoxide.

Preferably, the above preparation of the compound of formula (II) is carried out at a temperature of 80-150°C.

Preferably, the strong base used in the above preparation of the compound of formula (II) is potassium hydroxide or sodium hydroxide, preferably potassium hydroxide.

As in the case of the alkylation reaction, it is believed that the base reacts with the -OH group of the compound of formula (IV) to produce the salt, and this salt reacts with the compound of formula (V) to form the compound of formula (II). Sufficient strong base should, therefore, preferably be added to form the salt of the compound of formula (IV).

Many of the 3-hydroxy-4-nitro diphenylethers and the 3-alkoxy-4-nitro diphenylethers, produced according to the present invention, are known to exhibit herbicidal activity. For example, the herbicidal activity of many of the compounds produced according to the present invention is disclosed in the above identified U.K. and US patents.

Certain embodiments of the present invention will now be described in the following Examples.

### Examples

### Example 1:

### Preparation of 3-chloro-4-(3'-hydroxy-4'-nitro)phenoxybenzotrifluoride:

### (a). Preparation of 3-chloro-4-(4'-nitro)phenoxybenzotrifluoride.

To 2.78g (20mmol) of 4-nitrophenol in 40 ml of degassed dimethylsulfoxide in a flask was added quickly 2.61g (21 mmol) of 45% aqueous potassium hydroxide. The flask was evacuated and flushed with nitrogen several times before the solvent was removed by vacuum distillation. The flask was cooled to room temperature and recharged with 40 ml of dimethylsulfoxide and 3-chloro-4-fluorobenzotrifluoride (3.97g, 20 mmol) and then heated at 120°C overnight. The reaction was cooled to room temperature and the solvent was removed by vacuum distillation. The residue was partitioned between 10% HCl and ethyl acetate, the resulting layers were then separated, and aqueous extracted twice more using ethyl acetate. The organics were combined, dried over sodium sulfate, filtered and evaporated to dryness *in vacuo* to give a brown oil which was chromatographed (flash, 3:1 hexanes/ethyl acetate) to give 2.5g (7.9 mmol, 39%) of desired product as a light yellow solid. ¹H NMR (CDCl₃. 400 MHz) d 7.0 (dd, 2H, J=2.0, 6.8Hz), 7.2 (d, 1H, J=8.4 Hz), 7.6 (d, 1H, J=6.8 Hz), 7.9 (s, 1H), 8.2 (dd, 2H, J=2.0, 8.0 Hz).

### (b). Preparation of 3-chloro-4-(3'-hydroxy-4'-nitro)phenoxybenzotrifluoride:

To 30 ml of refluxing ammonia was added 5.3g (47 mmol) of solid potassium t-butoxide. This was followed by the dropwise addition over 15 minutes of 3.0g (9.4 mmol) of 3-chloro-4-(4'-nitro)phenoxybenzotrifluoride in 9 ml of tetrahydrofuran with 2.08 ml (10.4 mmol) of t-butylhydroperoxide. Once addition was complete the reaction was stirred for 1 hour at -33°C. The ammonia was evaporated and the residue partitioned between water and ethyl acetate. The layers were separated and aqueous extracted once more with ethyl acetate. The organics were combined, dried over sodium sulfate, filtered and evaporated to dryness *in vacuo* to give 1.5g of a red oil. Gas chromatography/Mass spectrometry indicates a 2.7:1 ratio of 3-chloro-4-(3'-hydroxy-4'-nitro)phenoxybenzotrifluoride:bis(2-chloro-4-trifluoromethylphenyl) ether. Chromatographed (flash 3:1 hexanes/ethyl acetate) 3-chloro-4-(3'-hydroxy-4'-nitro)phenoxybenzotrifluoride had a ¹H NMR (CDCl₃, 400 MHZ) d 6.45 (d, 1H, J=2.8 Hz), 6.55 (dd, 1H, J=2.8, 9.6 Hz), 7.2 (d, 1H, J=8.8 Hz), 7.6 (d, 1H. 9.2 Hz), 7.8 (s, 1H), 8.1 (d, 1H, 9.2 Hz).

## Claims

1. A process for preparing a compound of formula (I): 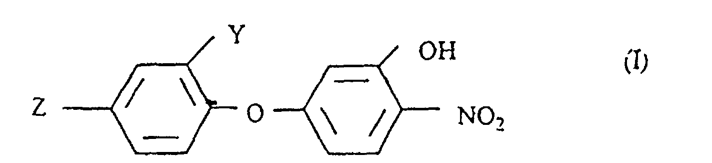 wherein Y is alkyl, halogen or a trihaloalkyl group, and Z is alkyl, halogen or a trihalo alkyl group,
the process comprising reacting a compound of formula (II): 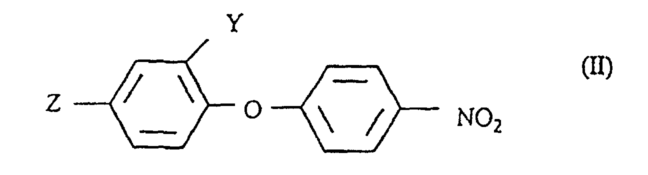 with a hydroperoxide in the presence of liquid ammonia and a strong base.

2. The process according to claim 1, wherein Y is chlorine.

3. The process according to claim 1, wherein Z is trifluoromethyl.

4. The process according to claim 1, wherein the strong base is potassium tertiary butoxide.

5. The process according to claim 1, wherein the hydroperoxide is tertiary butyl hydroperoxide.

6. The process according to claim 1, which comprises the additional step of alkylating the compound of formula (I) to produce the corresponding compound of formula (III): 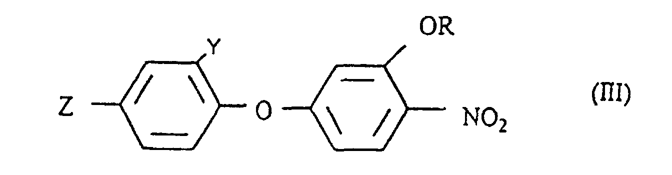 wherein R is an alkyl group or a substituted alkyl group and Z and Y are defined as in claim 1.

7. The process of claim 6 wherein
R is an alkyl group containing from one to twenty carbon atoms or a substituted alkyl group wherein said alkyl portion has from one to twenty carbon atoms:

8. The process according to claim 6, wherein the alkyl group is ethyl.

9. The process according to claim 6, wherein R is a substituted alkyl group wherein said alkyl portion has from one to twenty carbon atoms and is substituted with one or more substituents selected from: halo; hydroxy; alkoxy; alkoxycarbonyl; carboxyl; alkylcarbonyl; alkylthio; alkylsulfonyl; epoxy; aminocarbonyl; alkylamino; and alkylaminocarbonyl.

10. The process according to claim 6, wherein the alkylation comprises reacting the compound of formula (I) with a strong base and a compound having the formula RX, wherein R is an alkyl group or a substituted alkyl group, and X is halogen.

11. The process according to claim 10, wherein R is ethyl and X is chlorine, bromine or iodine.

12. The process according to claim 7, wherein Y is chlorine.

13. The process according to claim 7, therein Z is trifluoromethyl.

14. The process according to claim 6, wherein the strong base is selected from sodium hydroxide, sodium methoxide and potassium t-butoxide.

## Patentansprüche

1. Verfahren zur Herstellung einer Verbindung der Formel (I): 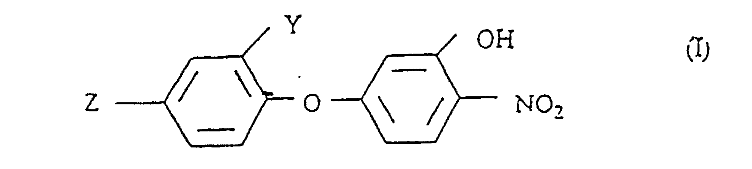 worin Y Alkyl, Halogen oder eine Trihaloalkylgruppe ist und Z Alkyl, Halogen oder eine Trihaloalkylgruppe ist,
worin das Verfahren das Umsetzen einer Verbindung der Formel (II): 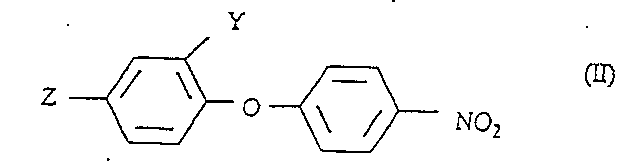 mit einem Hydroperoxid in der Gegenwart von flüssigem Ammoniak und einer starken Base umfasst.

2. Verfahren nach Anspruch 1, worin Y Chlor ist.

3. Verfahren nach Anspruch 1, worin Z Trifluormethyl ist.

4. Verfahren nach Anspruch 1, worin die starke Base Kaliumtertiärbutoxid ist.

5. Verfahren nach Anspruch 1, worin das Hydroperoxid Tertiärbutylhydroperoxid ist.

6. Verfahren nach Anspruch 1, umfassend den zusätzlichen Schritt des Alkylierens der Verbindung der Formel (I), um die entsprechende Verbindung der Formel (III) zu bilden: 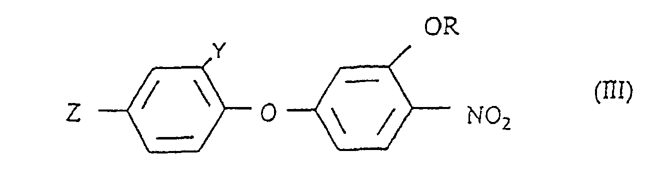 worin R eine Alkylgruppe oder eine substituierte Alkylgruppe ist und Z und Y wie in Anspruch 1 definiert sind.

7. Verfahren nach Anspruch 6, worin R eine Alkylgruppe mit von einem bis zwanzig Kohlenstoffatomen oder eine substituierte Alkylgruppe ist, worin der Alkylanteil von einem bis zwanzig Kohlenstoffatome aufweist.

8. Verfahren nach Anspruch 6, worin die Alkylgruppe Ethyl ist.

9. Verfahren nach Anspruch 6, worin R eine substituierte Alkylgruppe ist, worin der Alkylanteil von einem bis zwanzig Kohlenstoffatome aufweist und substituiert ist mit einem oder mehreren Substituenten, ausgewählt aus: Halogen; Hydroxy; Alkoxy; Alkoxycarbonyl; Carboxyl; Alkylcarbonyl; Alkylthio; Alkylsulfonyl; Epoxy; Aminocarbonyl; Alkylamino und Alkylaminocarbonyl.

10. Verfahren nach Anspruch 6, worin die Alkylierung das Umsetzen der Verbindung der Formel (I) mit einer starken Base und einer Verbindung mit der Formel RX umfasst, worin R eine Alkylgruppe oder eine substituierte Alkylgruppe ist und X Halogen ist.

11. Verfahren nach Anspruch 10, worin R Ethyl ist und X Chlor, Brom oder lod ist.

12. Verfahren nach Anspruch 7, worin Y Chlor ist.

13. Verfahren nach Anspruch 7, worin Z Trifluormethyl ist.

14. Verfahren nach Anspruch 6, worin die starke Base ausgewählt wird aus Natriumhydroxid, Natriummethoxid und Kalium-t-butoxid.

## Revendications

1. Procédé de préparation d'un composé de formule (I) : 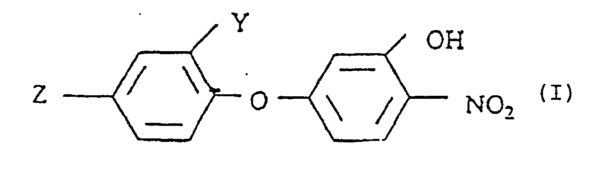 dans laquelle Y représente un atome d'halogène ou un groupe alkyle ou trihalogénoalkyle et Z représente un atome d'halogène ou un groupe alkyle ou trihalogénoalkyle, qui comprend la réaction d'un composé de formule (II) : 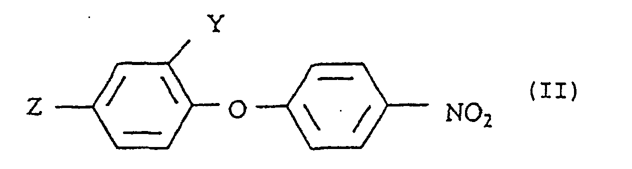 avec un hydroperoxyde, en présence d'ammoniac liquide et d'une base forte.

2. Procédé selon la revendication 1, dans lequel Y représente un atome de chlore.

3. Procédé selon la revendication 1, dans lequel Z représente le groupe trifluorométhyle.

4. Procédé selon la revendication 1, dans lequel la base forte est le tertiobutylate de potassium.

5. Procédé selon la revendication 1, dans lequel l'hydroperoxyde est l'hydroperoxyde de tertiobutyle.

6. Procédé selon la revendication 1, qui comprend l'étape supplémentaire consistant à alkyler le composé de formule (I) pour produire le composé correspondant de formule (III) : 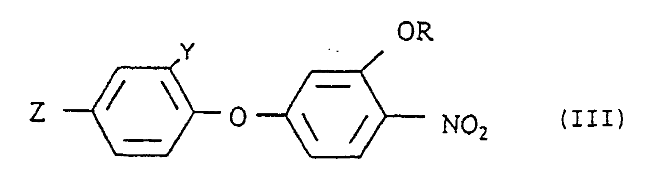 dans laquelle R représente un groupe alkyle ou un groupe alkyle substitué et Z et Y sont tels que définis dans la revendication 1.

7. Procédé selon la revendication 6, dans lequel R représente un groupe alkyle ayant 1 à 20 atomes de carbone ou un groupe alkyle substitué dont la partie alkyle a 1 à 20 atomes de carbone.

8. Procédé selon la revendication 6, dans lequel le groupe alkyle est le groupe éthyle.

9. Procédé selon la revendication 6, dans lequel R représente un groupe alkyle substitué dont la partie alkyle a 1 à 20 atomes de carbone et est substituée par un ou plusieurs substituants pris parmi les atomes d'halogène et les groupes hydroxyle, alcoxy, alcoxycarbonyle, carboxyle, alkylcarbonyle, alkylthio, alkylsulfonyle, époxy, aminocarbonyle, alkylamino et alkylaminocarbonyle.

10. Procédé selon la revendication 6, dans lequel l'alkylation comprend la réaction du composé de formule (I) avec une base forte et un composé de formule RX dans laquelle R représente un groupe alkyle ou un groupe alkyle substitué et X représente un atome d'halogène.

11. Procédé selon la revendication 10, dans lequel R représente le groupe éthyle et X représente un atome de chlore, de brome ou d'iode.

12. Procédé selon la revendication 7, dans lequel Y représente un atome de chlore.

13. Procédé selon la revendication 7, dans lequel Z représente le groupe trifluorométhyle.

14. Procédé selon la revendication 6, dans lequel on choisit la base forte parmi l'hydroxyde de sodium, le méthylate de sodium et le t-butylate de potassium.
